# EUROPEAN PATENT APPLICATION

(11) **EP 0 915 099 A1**
(43) Date of publication of application: **12.05.1999**
(21) Application number: 98120622.0
(22) Date of filing: 02.11.1998
(51) Int. Cl.: C07K 7/06, C07K 7/08, C07K 14/56, A61K 38/21

(54) **Antiparallel oligopeptides and hydrophobically complementary oligopeptides, matrices containing them and use thereof in the purification and detection of subtypes of alpha interferon**

(30) Priority: 04.11.1997 IT BO970655
(71) Applicant: ALFA WASSERMANN S.p.A., 65020 Alanno Scalo (Pescara) (IT)
(72) Inventor: Viscomi, Claudio Giuseppe, 40037 Sasso Marconi (Bologna) (IT); Bruno, Christiana, 44100 Ferrara (IT); Scapol, Lucia, 40037 Sasso Marconi (Bologna) (IT)
(74) Representative: Kraus, Walter, Dr.

(57) **Abstract**

Antiparallel oligopeptides and oligopeptides complementary from the hydrophobic point of view, whose sequences are opportunely established so that they show affinity towards some subtypes of alpha interferon, mainly for the subtype Hu-IFNα8, process for the purification of some subtypes of alpha interferon by means of the use of solid stationary phases made by inert matrices on which said oligopeptides are chemically bound and method for the detection of some subtypes of alpha interferon by means of the use of said oligopeptides in methods like ELISA and immunoblotting.

## Description

### BACKGROUND OF THE INVENTION

The human alpha interferon (Hu-IFNα) is made by a mixture of proteins produced by human leukocytes when they get in touch with extraneous agents like, for instance, viruses and bacteria. Thirteen different proteins have till now been identified, named subtypes of Hu-IFNα, which are listed from Allen G. and Diaz M.O., J. Interferon Res 14, 223-226, (1994) as: Hu-IFNα1, Hu-IFNα2, Hu-IFNα4, Hu-IFNα5, Hu-IFNα6, Hu-IFNα7, Nu-IFNα8, Hu-IFNα10, Hu-IFNα14, Hu-IFNα16, Hu-IFNα17, Hu-IFNα21 and Hu-IFNω, this latter also named type II Hu-IFNα. All these proteins are made by 166 aminoacids, except for Hu-IFNα2 which has 165 and Hu-IFNω which has 172 proteins. The Hu-IFNα subtypes show apparent molecular weights between 18,000 and 28,000 Daltons during the detection by means of the method of polyacrylamide gel electrophoresis with 15% of bisacrylamide under denaturing conditions and in presence of sodium dodecylsulfate (SDS-PAGE) and only the Hu-IFNα2, Hu-IFNα14 and Hu-IFNω subtypes are glycosylated.

Hu-IFNα exercises many biological activities that can be summarised in the antiviral, antiproliferative and immunostimulant activities (Baron S. et al., *Interferon, Principles and Medical Applications,* The University of Texas, Medical Branch of Galveston, Department of Microbiology, Galveston, TX, 1992). The Hu-IFNα subtypes distinguish themselves in such an ambit by showing differences sometimes strong, so that it happens that some of them possess a more remarkable antiviral activity, other an antiproliferative activity, while some even are antagonists of others in the immunostimulating activity [Viscomi C. G., et al., Medicinal Research Reviews 15, 445-478, (1995)].

On the basis of the mentioned biological activities it is evident how Hu-IFNα or the single subtypes can exercise remarkable pharmaco-therapeutic activities which can be summarised in the antiviral, antiproliferative and immunostimulating activities in the pathologies with a viral aetiology like hepatitis, papillomatosis, herpes infections, adenovirus and in the antiproliferative and immunostimulating activities in the treatment of tumor pathologies.

As a matter of fact Hu-IFNα and some of its single subtypes, mainly the recombinant form of IFNα2, are practically worldwide registered for the therapy of pathologies of the lymphatic and hemopoietic system, of the solid tumours and of pathologies with viral aetiology. In particular Hu-IFNα is prevailingly prescribed for B, C and D hepatitis, papillomatosis, hair cells leukemia, chronic myeloid leukaemia, multiple myeloma, renal carcinoma, non-Hodgkin lymphoma related to AIDS, malignant melanoma.

Its is important to have preparative systems suitable for selectively purifying the subtypes of Hu-IFNα and analytical systems suitable for selectively detecting their presence also in presence of complex proteic mixtures because of the wide clinical use of Hu-IFNα and of the possible differentiation of the pharmacological activity of the subtypes of Hu-IFNα.

At present the immunoaffinity chromatography based on the use of the mono and/or polyclonal antibodies is the more used technique to get the purification of the proteins. These antibodies are also widely used in systems of analytical detection and in clinical diagnostics, like the ELISA or SDS-PAGE immunoblotting systems.

The use of antibodies shows anyway a series of drawbacks that limit their application: difficulty of manufacturing at low costs, difficulty of handling molecules sensitive to the action of biological agents, like for instance the enzymes, of chemical agents, like for instance acids, bases, oxidizing agents and organic solvents and of physical agents, like for instance temperature and UV rays, risk of biological contaminations coming from patogens present in the cells of hybridomas or in the animals which produce antibodies, like for instance retroviruses and agents responsible for encephalopathies.

In the alternative it is known that peptides codified from a RNA sequence show affinity for those codified from the corresponding antiparallel sequence, named peptides AP, and can be used in the purification processes of the proteins as described in Italian Patent IT 1243419.

Moreover it is also known that the affinity between the peptides and the corresponding peptides AP can be further improved if the sequence of these latter is opportunely modified in such a way that the resulting hydropathic profiles are as much as possible specular with respect to the peptides on which the sequences are made [G. Fassina et al., J. Biol. Chem., 264, 11252, (1989)], the so modified peptides are named hydrophobic complementary peptides (IC).

The AP and IC peptides show remarkable advantages with respect to the mono and/or polyclonal antibodies because they keep the selectivity typical of the antibodies but, because of their chemical origin, they are more controllable from the analytical point of view, less costly, more speedy to be manufactured also in large amounts and safe from the point of view of the risk of biological contaminations.

The subtype Hu-IFNα8 resulted to be the compound endowed with the highest specific antiviral activity among all the Hu-IFNα subtypes and moreover it turned out that the transfer of the signal of the Hu-IFNα8 happens through a mechanism different from that of the other subtypes even if the receptor of the human leukocytes recognized from such subtype is identical to that of the other Hu-IFNα subtypes [Foster G. R. et al., J. Interferon Res., 16, 1027-1033, (1996)].

The high and specific antiviral activity and the different mechanism of induction make important to have large amounts of Hu-IFNα8, therefore processes and products which allow its extraction from whole mixtures of Hu-IFNα and its detection are of remarkable value. It has to be underlined that obtaining Hu-IFNα starting from Hu-IFNα mixtures is particularly important because Foster G. R. et al., J. Interferon Res., 16, 1027-1033, (1996) showed that the corresponding Hu-IFNα8 products obtained through the technology of the recombinant DNA showed biological activities lower than those of the natural subtype.

### DESCRIPTION OF THE INVENTION

The present invention refers to oligopeptides whose sequence has been defined in such a manner that they result to be antiparallel oligopeptides, peptides AP, or hydrophobic complementary oligopeptides, peptides IC, of the aminoacids' sequences 142-151 of Hu-IFNα and Hu-IFNω, hereinafter respectively indicated as IFNα-AP, IFNω-AP, IFNα-IC and IFNω-IC.

Notwithstanding the aminoacids' sequence 142-151 of Hu-IFNα is identical in all the subtypes of Hu-IFNα, the IFNα-AP, IFNω-AP, IFNα-IC and IFNω-IC peptides turn out unexpectedly to have affinity, both in solution and bound to solid supports, only for some subtypes of Hu-IFNα, mainly for the subtype IFNα8. Therefore they can be used, when bound to suitable matrices on solid supports, in affinity chromatography for the separation and purification of the subtype Hu-IFNα8. Moreover, when conveniently functionalized, they can be used for the specific detection of Hu-IFNα8 in analytical techniques like the detection of compounds splitted in SDS-PAGE and transferred on nitrocellulose sheets (SDS-PAGE-blotting) or the detection of compounds immobilized on solid supports (enzyme linked immuno sorbent assay or ELISA). Therefore the matrices on solid supports for the affinity chromatography to which the IFNα-AP, IFNω-AP, IFNα-IC and IFNω-IC oligopeptides are linked and the use thereof in the separation and purification of the Hu-IFNα8 protein are a further object of the invention.

The solid supports preferred in carrying out the invention are made by polymeric resins based on acrylamide or agarose.

Moreover the IFNα-AP, IFNω-AP, IFNα-IC and IFNω-IC oligopeptides functionalized with biotine and the use thereof in the analysis of the mixtures of Hu-IFNα and in the detection of the Hu-IFNα8 in said mixtures according to the procedures scheduled from the detection methods ELISA and SDS-PAGE-blotting are a further object of the invention.

Lastly also the pharmaceutical compositions containing the subtype Hu-IFNα8 obtained from processes of separation and purification which foresee the use of matrices on solid supports for affinity chromatography containing said oligopeptides constitute scope of the present invention.

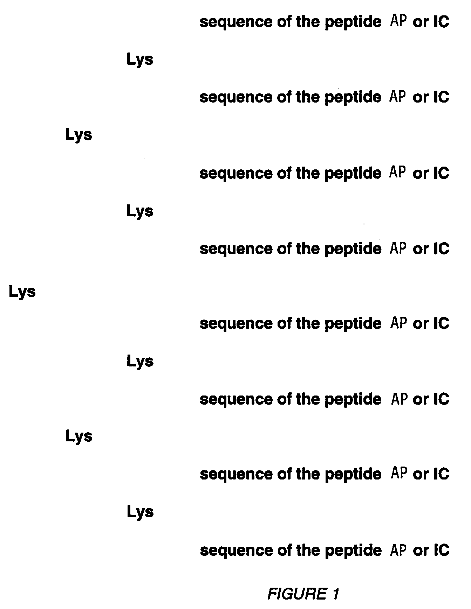

The peptides AP object of the present invention show the following sequences:
1. IFNα-AP: H-Gly-Ser-His-Asp-Phe-Cys-Ser-Asp-Asn-Leu-OH
2. IFNω-AP: H-Gly-Phe-His-Asp-Phe-His-Ser-Asp-Asn-Phe-OH
   Two of the peptides IC object of the present invention show the following sequences, established by means of a process of successive approximations, each time changing the residues until the better hydropathic complementarity is obtained, on the basis of the values of hydrophobicity of the aminoacids reported by Kyte J. and Doolittle R. F. [J. Mol. Biol 157, 105-132, (1982)]:
3. IFNα-IC-KD: H-Ile-Arg-Ser-Tyr-Pro-Leu-Phe-His-Pro-Glu-Val-Phe-Tyr-Arg-OH
4. IFNω-IC-KD: H-Phe-Arg-Ala-Gly-Thr-Ala-Gly-Gln-Met-Tyr-Ala-Thr-Gly-Arg-OH
   The remaining two peptides IC object of the present invention, prepared on the basis of the values of hydrophobicity reported by Chothia C. [J. Mol. Biol., 105, 1-14, (1976)], show the following sequences:
5. IFNα-IC-Ch: H-Val-Glu-Gly-Ser-His-Asp-Phe-Ser-Ser-Lys-Gly-Cys-Gly-Arg-OH
6. IFNω-IC-Ch: H-Gly-Gln-Gly-Phe-His-Asp-Phe-Ser-Ser-Lys-Ser-Leu-Gly-Arg-OH
   The peptides have been synthesized in octameric form on a branched chain of 7 residues of lysine (figure 1) by means of the synthesis on solid phase according to the procedures described by Atherton E. and Sheppard R. C., *Solid phase peptide* synthesis, a practical approach, published by IRL PRESS, Oxford, New York and Tokyo, pages 25-37 (1989).

Each of the peptides from number 1 to number 6, at the end of the synthesis, has been linked to a solid support useful for affinity chromatographies, preferably made by a polymeric resin based on acrylamide or agarose, and each matrix functionalized in this way has been packed on a chromatographic column on which, at a neutral pH, a mixture of Hu-IFNα having a specific activity between 1 e 100 x 10⁵ international units for each milligram of total proteins (IU/mg) and with a Hu-IFNα concentration between 0.1 and 5 x 10⁶ IU has been pressed through. The column after the charge has been washed with a solution 0.1 M of dibasic sodium phosphate and 0.5 M of sodium chloride at neutral pH and the material remained linked to the solid support after the washings has been eluted with an acidic aqueous solution at pH 2, for instance an aqueous solution containing 0.1% of trifluoroacetic acid or a 0.1 M aqueous solution of citric acid.

The product eluted in such way from the column has then been analyzed in SDS-PAGE with silvery coloration and in immunoblotting by using monoclonal antibodies specific for Hu-IFNα and the products eluted from said chromatography showed a molecular weight between 27 and 28 kDaltons, specific for the subtype Hu-IFNα8, they were of interferon origin because they were recognized from antibodies specific for Hu-IFNα, they had an antiviral activity typical of Hu-IFNα, detected by measuring the inhibition of the cytopathic effect of the VSV virus on MDBK cells according to the method of Armstrong J. A. [Meth. in Enzymol., 78 381-387, (1981)] and they showed a peak corresponding with a standard of Hu-IFNα8 in the retention time in the high pressure liquid chromatography in reverse phase (RP-HPLC).

Hu-IFNα8 has been further purified by elution on a molecular exclusion column with molecular cut at 10,000 Daltons and subsequent lyophilization of the eluate. Hu-IFNα8 so obtained can be advantageously used for the preparation of pharmaceutical compositions useful for the treatment of viral diseases and of tumor affections.

Pharmaceutical compositions for parenteral use are made by dissolving from 0.1 to 20 millions IU/ml of Hu-IFNα8 in sterile physiological aqueous solutions containing human serum albumin as proteins stabilizer.

Moreover the peptides from number 1 to number 6 have been functionalized with the biotine and the resulting products have been used for the analysis of mixtures of Hu-IFNα separated in SDS-PAGE and transferred on a nitrocellulose sheet. The bands on which the peptides were linked have been evidenced by using a solution containing suitable compounds conjugated to enzymes like, for instance, the streptavidine conjugate with the peroxidase, which colour the band as a result of a colourimetric reaction.

The peptides from number 1 to number 6 functionalized with biotine have also been used for the detection of the presence of Hu-IFNα8, when this is immobilized alone or in admixtures of Hu-IFNα in plates with tubes such as those normally used for the tests of ELISA type. Solutions containing suitable compounds conjugate to enzymes as in the previous case like, for instance, the streptavidine conjugate with the peroxidase, have been used for colouring the tubes containing Hu-IFNα8.

The following examples report, with an illustrative but not limitative scope, the preparation and use of the AP and IC peptides according to the invention and pharmaceutical compositions containing Hu-IFNα8.

### EXAMPLE 1

### Synthesis of the IFNα-AP peptide (sequence of the monomer: H-Gly-Ser-His-Asp-Phe-Cys-Ser-Asp-Asn-Leu-OH)

The synthesis of the peptide has been carried out in solid phase by treating 2 g of polyacrylamide resin with 20 ml of 1,6-hexanediamine at 50°C for 12 hours. The resulting resin has been put on a column having a diameter of 3 cm and a porous screen at the terminal extremity and has been washed with N,N-dimethylformamide during 60 minutes at 4 ml/min flow. Such a flow has been kept constant during all the subsequent operations.

The resin has then been functionalized with 0.6 mM of 1 -hydroxybenzotriazole in 4 ml of N,N-dimethylformamide and 0.6 mM of 2,4,5-trifluorophenyl-4-hydroxymethylphenoxyacetate in 4 ml of N,N-dimethylformamide. The resin treated in such a way showed a functionalization equal to 0.1 milliequivalents/g.

All the aminoacid residues have been introduced with the α-amino group protected with the fluorenylmethoxycarbonyle. The esters of the pentafluorophenol of all the aminoacids (0.3 mM in 4 ml of N,N-dimethylformamide) have been used for the peptidic condensation in the presence of 0.3 mM of 1 -hydroxybenzotriazole.

The functions in the side chain of the residues of the aspartic acid have been protected with tert-butylester, those of histidine and of lysine with tert-butyloxycarbonyle, those of the serine with tert-butylether, that of cysteine with tert-butylthioether. The active esters have been dissolved in N,N-dimethylformamide immediately before their addition to the resin and the reaction of acylation has been carried out at room temperature during 60 minutes under recycling conditions.

Between each acylation reaction and the subsequent one the resin has been washed with N,N-dimethylformamide during 10 minutes and then it has been contacted with a 20% solution of piperidine in N,N-dimethylformamide during 10 minutes in order to remove the protecting group fluorenylmethoxycarbonyle and lastly it has been washed again during 10 minutes with N,N-dimethylformamide. The completion of the reaction has been detected by means of the ninidrine test [Kaisen E. et al., Anal. Biochem., 34, 595, (1970)] and the trinitrobenzenesulfonic acid test [Hancock W. S. et al., Anal. Biochem., 71, 261, (1976)] after each reaction of acylation.

The pentafluorophenolester of the aminoacid was again added in the same amounts used before, by repeating the test for checking the completeness of the reaction, in the case in which the reaction was not completed 30 minutes after the addition of the aminoacid derivative and free amino groups were present. The resin has been treated with 10 ml of a 95% solution of trifluoroacetic acid and 1% solution of anisole in dichloromethane after the end of the addition of the aminoacid residues in the appropriate order, so obtaining the releasing of the peptide from the resin and contemporaneously the removal of all the protective groups from the side chains.

The solvents have been removed by evaporation under vacuum and the peptide has been treated with 5 ml of water and partially purified by means of a molecular exclusion chromatography by using a resin having a molecular cut at 2000 Daltons (40 x 2 cm column, flow equal to 0.5 ml/min. and 0.05 M aqueous solution of ammonium chloride as eluent).

The peak containing the peptide has been freeze-dried, taken with 20 ml of a 0.01 M aqueous solution of ammonium bicarbonate and chromatographed on an anionic exchange column, diethylaminoethyl Sephadex, by using an aqueous solution of ammonium bicarbonate as eluent with a linear gradient from 0.01 to 0.5 M in 10 hours (15x1 cm column, flow equal to 0.5 ml/min.). The fractions containing the main product have been collected and freeze-dried. The residue has been taken in 5 ml of a 0.1% aqueous solution of trifluoroacetic acid and has been further purified by means of an inverse phase chromatography with an acetonitrile gradient from 0% to 90% of acetonitrile in a 0.1% aqueous solution of trifluoroacetic acid in one hour (250x10 mm Vydac C18 column, flow equal to 3 ml/min.) by collecting the main peak showing the following aminoacids' analysis: Gly, 7.83 (8.00); Ser, 14.75 (16.00); His, 7.52 (8.00); Asx, 25.60 (24.00); Phe, 7.76 (8.00); Cys, 6.85 (8.00); Leu, 8.00 (8.00); Lys, 7.50 (7.00).

The product has been analyzed also in capillary electrophoresis (capillar made by melted silica 47 cm long, 40 cm length at the UV detector, 75 µm diameter, 20 kV constant voltage with an eluent made of a 0.1 M aqueous solution of phosphoric acid and monobasic sodium phosphate at pH 2.5) obtaining a time of electrophoretic migration equal to 8.33 minutes.

### EXAMPLE 2

### Synthesis of the IFNωIC-Ch peptide (monomer sequence: H-Gly-Gln-Gly-Phe-His-Asp-Phe-Ser-Ser-Lys-Ser-Leu-Gly-Arg-OH)

The synthesis and purification of the peptide have been carried out according to methods equal to those described in example 1.

The side chain functions of the residues of the aspartic and glutamic acids have been protected with the tert-butylester, those of the lysine and histidine with the tert-butoxy carbonyle, those of the serine with the tert-butylether and those of the arginine with the 4-methoxy-2,3,6-trimethylbenzenesulfonyle.

The following values come out from the analysis of the aminoacids:
Gly, 22.99 (24.00); Ser, 21.78 (24.00); His, 6.78 (8.00); Asx, 7.34 (8.00); Phe, 16.50 (16.00); Arg, 6.85 (8.00); Leu, 8.00 (8.00); Lys,17.60 (15.00); Glx, 8.78 (8.00).

The product has also been analyzed by capillary electrophoresis under modalities equal to those described in example 1 obtaining an electrophoretic migration time equal to 8.99 minutes.

### EXAMPLE 3

### Preparation of a resin functionalized with the IFNα-AP peptide

300 Milligrams of Sepharose Fast-Flow CN-Br resin (Pharmacia) have been swollen and washed with 100 ml of a 1 mM aqueous solution of hydrochloric acid at 4°C.

10 milligrams of IFNα-AP peptide dissolved in 2 ml of a 0.1 M sodium chloride and 0.5 M sodium bicarbonate aqueous solution at pH 8.3 have been added to the resin and stirred overnight at 4°C; the resin has then been decanted and washed with the same aqueous solution. The unreacted functions of the resin have been blocked by means of two consecutive cycles of washings made with 10 ml of a 1 M aqueous solution of ethanolamine at pH 8 and then with a 0.1 M sodium acetate and 0.5 M sodium chloride aqueous solution brought to pH 4 by means of a 0.1 M aqueous solution of hydrochloric acid.

Lastly the resin has been washed with a 0.15 M sodium chloride and 0.15 M dibasic sodium phosphate (PBS) aqueous solution at pH 7.2 and stored in PBS containing 0.02 % of sodium azide.

### EXAMPLE 4

### Purification of Hu-IFNα8

One milliliter of the resin described in Example 3, packed on a chromatographic column (⌀ 0.5 cm) has been washed with 10 ml of PBS. One milliliter of a solution containing 6 x 10⁶ IU of Hu-IFNα coming from a colture of leukocytes stimulated to produce Hu-IFNα by the Sendai virus and partially purified according to what described by Cantell K. et al., *Methods in Enzymol*., 78, 29, (1981) e 78, 499, (1981) has been filled in the column. The stationary phase has been washed with 10 ml of a 0.2 M sodium chloride and 0.15 M dibasic sodium phosphate solution and with 10 ml of a 0.5 M sodium chloride and 0.15 M dibasic sodium phosphate solution and lastly with PBS containing 10% of ethylene glycol. The material still adsorbed on the resin has been eluted from the column with 5 ml of a 0.1 M citric acid and 0.2 M sodium chloride aqueous solution at pH 2 and brought to pH 7.2 with a 0.1 M aqueous solution of sodium hydroxide. The product eluted has been analyzed on SDS-PAGE on a gel prepared at the 10% acrylamide concentration and with the silvery coloration, giving only one band at 28 kDaltons corresponding to a Hu-IFNα8 standard in the electrophoretic migration. In a parallel SDS-PAGE analysis, without silvery coloration, the band has been transferred on a nitrocellulose sheet and has reacted with a monoclonal antibody showing affinity to Hu-IFNα8 (NK-2, Cell Tech, UK). The material eluted from the column showed an antiviral activity equal to 40,000 IU/ml checked according to the method of the inhibition of the cytopathic activity.

10 Ml of product eluted have been further purified by filling them on a molecular exclusion column with molecular cut at 10,000 Daltons, 45 cm high and with a 2.5 cm diameter. The solution has been eluted with a flow speed equal to 3 ml/min by using as eluent an aqueous solution containing 0.3 mg/ml of monobasic sodium phosphate, 1.2 mg/ml of dibasic sodium phosphate and 8.0 mg/ml of sodium chloride. The eluate has then been freeze-dried obtaining highly pure Hu-IFNα8.

### EXAMPLE 5

### Pharmaceutical composition containing Hu-IFNα8

Vials for parenteral use containing 1 x 10⁶ IV of Hu-IFNα8 have been prepared by dissolving 1 x 10⁹ IU of Hu-IFNα8, obtained according to the method described in example 4, in 900 ml of bidistilled apyrogenous water containing 7.5 g of a 20% solution of human serum albumin. Sodium chloride, monobasis sodium phosphate and dibasic sodium phosphate have been added to this solution in such amounts that in the resulting solution 8.0 mg/ml of sodium chloride, 1.2 mg/ml of monobasic sodium phosphate and 0.3 mg/ml of dibasic sodium phosphate are present. Finally the solution has been brought to a volume equal to 1000 ml by means of apyrogenous bidistilled water containing the above mentioned amounts of salts, filtered on sterilizing filters and shared into 1000 vials containing 1 ml for parenteral use.

### EXAMPLE 6

### Preparation of IFNα-AP derivatives with biotine (Biot-IFNα-AP)

400 Microliters of a solution made dissolving 2 mg of biotinyl-ε-aminocaproate of N-hydroxysuccinimide in 1 ml of a 1:1 methanol/water mixture have been added to 2.5 mg of IFNα-AP peptide dissolved in 1 ml of a 20 mM aqueous solution of sodium acetate at pH 6.

The mixture has been kept under stirring at room temperature during 5 hours, then it has been filtered with a 0.45 µm filter and has been passed across a chromatographic column containing the IFNα-AP resin described in example 3 and packed as described in example 4. The material still adsorbed on the column after the washings with 10 ml of a 0.5 M sodium chloride and 0.15 M dibasic sodium phosphate solution, has been eluted with 10 ml of a 1% aqueous solution of trifluoroacetic acid. The resulting product Biot-IFNα-AP has been freeze-dried and taken with 2 ml of 0.01 M aqueous solution of dibasic sodium phosphate at pH 7.2 and analyzed in capillary electrophoresis (capillar made by melted silica 47 cm long, length 40 cm at the UV detector, 75 µm diameter, 20 kV constant voltage, 0.1 M aqueous solution of monobasic sodium phosphate at pH 2.5 as eluent) obtaining a sole peak having a migration time equal to 7.90 minutes.

### EXAMPLE 7

### Use of Biot-IFNα-AP in type ELISA analysis

30,000 IU of Hu-IFNα in 0.1 ml of a 50 mM sodium carbonate-bicarbonate aqueous solution buffered at pH 9.6 have been put into tubes of a plate for ELISA (Pierce) and have been incubated at 37°C during two hours. Then three washings with 0.1 ml of PBS containing 0.05% of Tween 20 and three washings with 0.2 ml of 3% bovine albumin in water have been carried out, lastly a further washing with 0.1 ml of PBS containing 0.05% of Tween 20 has been carried out. 0.1 Ml of the preparation of Biot-IFNα-AP described in example 6 diluted 1:20 with a 50 mM aqueous solution of sodium carbonate-bicarbonate buffered at pH 9.6 have been added into the tubes. The mixture has been incubated overnight at 4°C and then three washings with 0.1 ml of PBS containing 0.05% of Tween 20 have been carried out and the mixture has been incubated during two hours with 0.1 ml of peroxidated streptavidine diluted 1:1000 (Boehringer). The plate has been washed with PBS and coloured with Blue-POD (Boehringer) reading at 370 nm at the spectrophotometer. The coloration has been blocked after about 15 minutes, in function of the value of the reading at 370 nm, by means of a 0.1 M aqueous solution of sulfonic acid and the plate has been read at 450 nm.

Other tubes have been separated with the same procedure as that above shown with the difference that 0.1 ml of an aqueous solution containing 1 mg/ml of human albumin have been added instead of the solution containing Hu-IFNα. From the reading of the plate at 450 nm it has come out that Biot-IFNα-AP selectively recognizes Hu-IFNα mixtures containing Hu-IFNα8.

### EXAMPLE 8

### Use of Biot-IFNα-AP in blotting type analysis

Following the procedures described by Hames B. D. and Rickwood D. on "Gel electrophoresis of proteins. A practical approach." published by IRL Press, Oxford, New York, Tokyo, 1990, a gel for SDS-PAGE has been prepared containing 15% of acrylamide and 0.8% of bis-acrylamide and after the electrophoretic run has been effected, the bands have been transferred on a nitrocellulose sheet. Two samples containing 5 µl of an aqueous solution containing 1x10⁶ IU/ml of Hu-IFNα comprising also the Hu-IFNα8 subtype have been charged on the gel in a spaced manner charging two samples of 5 µl of a solution containing 1 mg/ml of human albumin as control in a spaced manner in respect to the preceding two samples.

The sheet has been cut after the transfer of the bands on the nitrocellulose in such a manner that each of the two portions included a sample of Hu-IFNα and one of albumin. The two portions have been saturated with a 3% aqueous solution of bovine albumin during two hours at room temperature and then have been washed with PBS containing 0.01% of Tween 20. The two portions have then been incubated in separated manner overnight at 4°C, one with 10 ml of the solution of Biot-IFNα-AP coming from example 6 diluted 1:20, the other with a murine monoclonal antibody showing affinity to Hu-IFNα8 (NK-2, Cell Tech, UK) diluted 1:1000.

After three washings with the PBS solution containing 0.01% of Tween 20, the portion of the nitrocellulose sheet that had been in contact with the Biot-IFNα-AP peptide has been incubated with peroxidated streptavidine diluted 1:1000, while the portion that had been in contact with the monoclonal antibody has been incubated with an antibody specific for IgG of mouse conjugated with the peroxidase. After the development of the colour a sole identical band has been identified in correspondence of the charge of Hu-IFNα in both the portions of the sheet while no coloration was visible in correspondence of the charge of human albumin. The revealed band showed an electrophoretic migration corresponding to 28 kDaltons characteristic of Hu-IFNα8.

## Claims

1. Antiparallel oligopeptides showing affinity for the Hu-IFNα8 protein.

2. Oligopeptide according to claim 1 characterized by the sequence: H-Gly-Ser-His-Asp-Phe-Cys-Ser-Asp-Asn-Leu-OH.

3. Oligopeptide according to claim 1 characterized by the sequence: H-Gly-Phe-His-Asp-Phe-His-Ser-Asp-Asn-Phe-OH.

4. Hydrophobic complementary oligopeptides showing affinity for the Hu-IFNα8 protein.

5. Oligopeptide according to claim 4 characterized by the sequence: H-Ile-Arg-Ser-Tyr-Pro-Leu-Phe-His-Pro-Glu-Val-Phe-Tyr-Arg-OH.

6. Oligopeptide according to claim 4 characterized by the sequence: H-Phe-Arg-Ala-Gly-Thr-Ala-Gly-Gln-Met-Tyr-Ala-Thr-Gly-Arg-OH.

7. Oligopeptide according to claim 4 characterized by the sequence: H-Val-Glu-Gly-Ser-His-Asp-Phe-Ser-Ser- Lys-Gly-Cys-Gly-Arg-OH.

8. Oligopeptide according to claim 4 characterized by the sequence: H-Gly-Gln-Gly-Phe-His-Asp-Phe-Ser-Ser-Lys-Ser-Leu-Gly-Arg-OH.

9. Matrices on solid supports for affinity chromatography for the separation and purification of the Hu-IFNα8 protein containing an oligopeptide according to claims 1 to 3.

10. Matrices on solid supports for affinity chromatography for the separation and purification of the Hu-IFNα8 protein containing an oligopeptide according to claims 4 to 8.

11. Matrices according each of claims 9 and 10 characterized in that the solid support is made by a polymeric resin based on acrylamide or agarose.

12. Oligopeptides according to claims 1 to 8 functionalized with the biotine.

13. Use of oligopeptides according to claim 12 in the analysis of Hu-IFNα mixtures.

14. Use of oligopeptides according to claim 12 in the detection of the Hu-IFNα8 within Hu-IFNα mixtures.

15. Pharmaceutical compositions containing Hu-IFNα8 separated and purified on the matrices on solid supports for affinity chromatography according to claims 9 to 11.

16. Pharmaceutical compositions according to claim 15 characterized in that they are made of vials for parenteral use containing from 0.1 to 20 x 10⁶ IU/ml of Hu-IFNα8 in sterile physiologic aqueous solutions containing human serum albumin.


SEQUENCE LISTING

(1) GENERAL INFORMATION:

(i) APPLICANT:
(A) NAME: ALFA WASSERMANN S.p.A.
(B) STREET: Contrada Sant'Emidio s.n.c.
(C) CITY: Alanno
(D) STATE: Pescara
(E) COUNTRY: Italy
(F) POSTAL CODE (ZIP): 65020

(ii) TITLE OF INVENTION: Antiparallel oligopeptides and
hydrophobically complementary oligopeptides, matrices
containing them and use thereof in the purification and
detection of subtypes of alpha interferon

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 10 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 1:

Gly Ser His Asp Phe Cys Ser Asp Asn Leu
1               5                   10

(2) INFORMATION FOR SEQ ID NO: 2:

(i) SEQUENCE CHARACTERISTICS:
(A) LENGTH: 10 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 2:

Gly Phe His Asp Phe His Ser Asp Asn Phe
1               5                   10

(2) INFORMATION FOR SEQ ID NO: 3:

(i) SEQUENCE CHARACTERISTICS:
(A) LENGTH: 14 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 3:

Ile Arg Ser Tyr Pro Leu Phe His Pro Glu Val Phe Tyr Arg
1               5                   10

(2) INFORMATION FOR SEQ ID NO: 4:

(i) SEQUENCE CHARACTERISTICS:
(A) LENGTH: 14 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 4:

Phe Arg Ala Gly Thr Ala Gly Gln Met Tyr Ala Thr Gly Arg
1               5                   10

(2) INFORMATION FOR SEQ ID NO: 5:

(i) SEQUENCE CHARACTERISTICS:
(A) LENGTH: 14 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 5:

Val Glu Gly Ser His Asp Phe Ser Ser Lys Gly Cys Gly Arg
1               5                   10

(2) INFORMATION FOR SEQ ID NO: 6:

(i) SEQUENCE CHARACTERISTICS:
(A) LENGTH: 14 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 6:

Gly Gln Gly Phe His Asp Phe Ser Ser Lys Ser Leu Gly Arg
1               5                   10